# EUROPEAN PATENT APPLICATION

(11) **EP 2 796 455 A1**
(43) Date of publication of application: **29.10.2014**
(21) Application number: 12859632.7
(22) Date of filing: 10.12.2012
(51) Int. Cl.: C07D 317/24, C07C 309/65, C07C 309/71

(54) **METHOD FOR PRODUCING SULFONATE**

(30) Priority: 19.12.2011 JP 2011277697; 19.12.2011 JP 2011277698; 19.12.2011 JP 2011277699; 19.12.2011 JP 2011277700; 19.12.2011 JP 2011277701; 19.12.2011 JP 2011277702
(71) Applicant: Sumitomo Chemical Co., Ltd, Chuo-ku, Tokyo 104-8260 (JP)
(72) Inventor: TANAKA, Yohei, Osaka-shi Osaka 555-0021 (JP); YASUOKA, Junichi, Osaka-shi Osaka 541-8550 (JP); AIKAWA, Toshiaki, Osaka-shi Osaka 555-0021 (JP); IKEMOTO, Tetsuya, Osaka-shi Osaka 555-0021 (JP)
(74) Representative: Nieuwenhuys, William Francis
(86) International application number: PCT/JP2012/082588
(87) International publication number: WO 2013/094546

(57) **Abstract**

A sulfonate represented by formula (7): 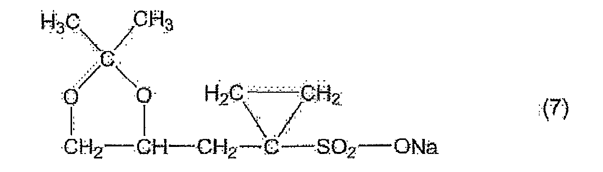 that is useful as a precursor for a chemotherapeutic medicament can be produced by a method for producing the sulfonate represented by formula (7) which comprises a step of reacting a sulfonic acid ester represented by formula (6): 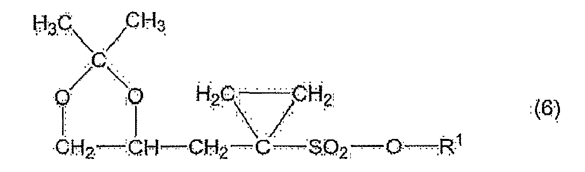 wherein R¹ represents a C₄ to C₆ branched alkyl group,
with sodium iodide or sodium hydroxide to obtain the sulfonate represented by formula (7).

## Description

### TECHNICAL FIELD

The present invention relates to a method for producing a sulfonate and the like.

### BACKGROUND ART

A sulfonate represented by formula (7A): 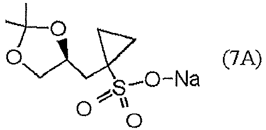 is a compound useful as a precursor for a chemotherapeutic medicament (see, for example, WO 2009/018238 and WO 2011/009541).

In Example 8 (Step A) and Example 9 of WO 2011/009541, a method for producing the sulfonate represented by formula (7A) according to the scheme shown below is described.

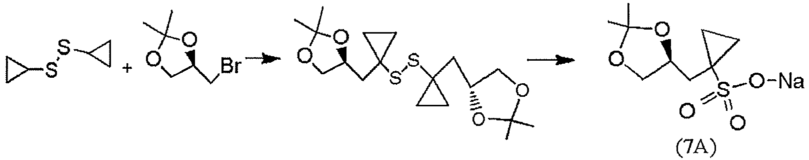

### SUMMARY OF THE INVENTION

The present invention provides a new method for producing a sulfonate represented by formula (7) shown in the following scheme and an intermediate used in the production method.

The summary of the production method of the present invention is represented by the following scheme: 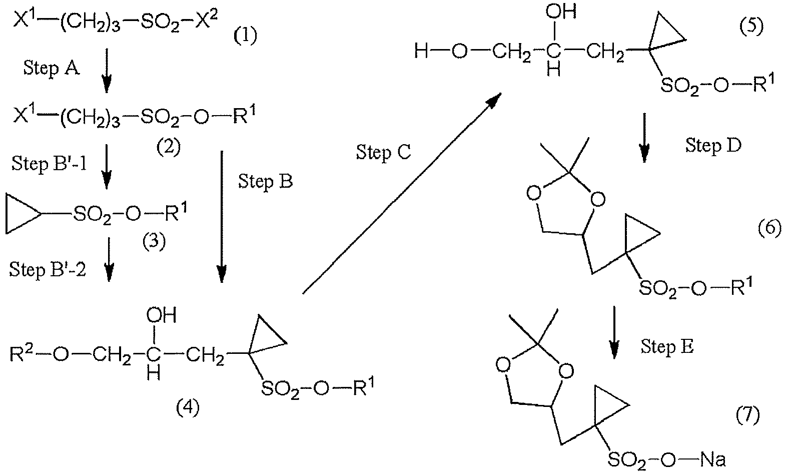 wherein X¹ and X² each independently represent a chlorine atom, a bromine atom or an iodine atom, and R¹ represents a C₄ to C₆ branched alkyl group.

More specifically, according to the present invention, the sulfonate represented by formula (7) can be produced from a sulfonic acid ester represented by formula (6) according to Step E. The sulfonic acid ester represented by formula (6) can be produced from a sulfonic acid ester represented by formula (5) according to Step D, and the sulfonic acid ester represented by formula (5) can be produced from a sulfonic acid ester represented by formula (4) according to Step C. The sulfonic acid ester represented by formula (4) can be produced from a sulfonic acid ester represented by formula (2) according to Step B or Step B'-1 and Step B'-2. The sulfonic acid ester represented by formula (2) can be produced from a sulfonyl halide represented by formula (1) according to Step A.

Furthermore, according to the present invention, the sulfonate represented by formula (7): 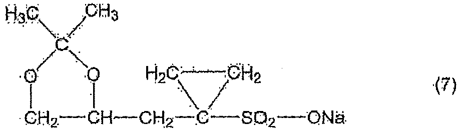 can be produced from the sulfonyl halide represented by formula (1), by a route including:
(Step A)
   a step of reacting a sulfonyl halide represented by formula (1) :

   **X¹- (CH₂)₃-SO₂-X²** **(1)**

   wherein X¹ and X² each independently represent a chlorine atom, a bromine atom or an iodine atom,
   with an alcohol represented by formula (11):

   **R¹-OH** (**11**)

   wherein R¹ represents a C₄ to C₆ branched alkyl group,
   in the presence of a base to obtain a sulfonic acid ester represented by formula (2):

   **X¹- (CH₂) ₃-SO₂-O-R¹** **(2)**

   wherein X¹ represents a chlorine atom, a bromine atom or an iodine atom, and R¹ represents a C₄ to C₆ branched alkyl group,
(Step B)
   a step of reacting the sulfonic acid ester represented by formula (2) with a base to obtain a cyclopropanesulfonic acid ester represented by formula (3): 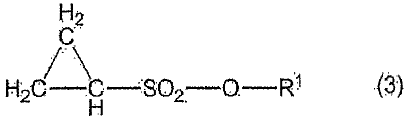 wherein R¹ represents a C₄ to C₆ branched alkyl group, (Step B' -1), and
   reacting the cyclopropanesulfonic acid ester with an epoxy compound represented by formula (31): 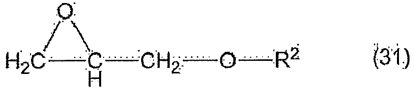 wherein R² represents a protecting group of a hydroxyl group, in the presence of a base to obtain a sulfonic acid ester represented by formula (4): 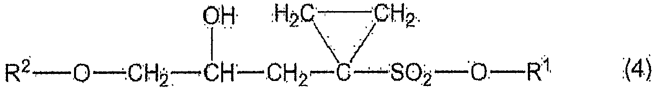 wherein R¹ represents a C₄ to C₆ branched alkyl group and R² represents a protecting group of a hydroxyl group, (Step B'-2), or
   a step of reacting the sulfonic acid ester represented by formula (2) with the epoxy compound represented by formula (31) in the presence of a base to obtain the sulfonic acid ester represented by formula (4),
(Step C)
   a step of deprotecting the sulfonic acid ester represented by formula (4) to obtain a sulfonic acid ester represented by formula (5): 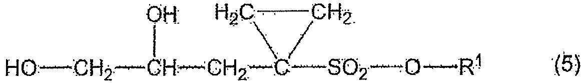 wherein R¹ represents a C₄ to C₆ branched alkyl group,
(Step D)
   a step of reacting the sulfonic acid ester represented by formula (5) with a 2,2-dialkoxypropane represented by formula (51): 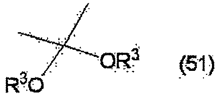 wherein R³ represents a C₁ to C₆ alkyl group,
   in the presence of an acid to obtain a sulfonic acid ester represented by formula (6): 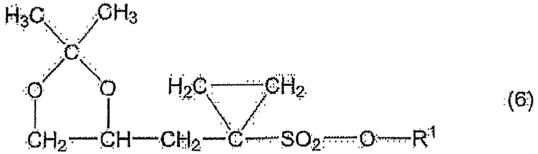 wherein R¹ represents a C₄ to C₆ branched alkyl group, and
(Step E)
   a step of reacting the sulfonic acid ester represented by formula (6) with sodium iodide or sodium hydroxide to obtain a sulfonate represented by formula (7): 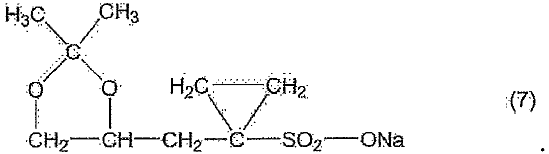 X¹ represents a chlorine atom, a bromine atom or an iodine atom, and preferably a chlorine atom.

The C₄ to C₆ branched alkyl group represented by R¹ is, for example, isobutyl, sec-butyl, tert-butyl, isopentyl, neopentyl, 1-ethylpropyl, isohexyl, 1,1-dimethylbutyl, 2,2-dimethylbutyl, 3,3-dimethylbutyl or 2-ethylbutyl, and preferably isobutyl or neopentyl.

Examples of the protecting group of a hydroxyl group represented by R² include benzyl ether type protecting groups such as benzyl group, 1-phenylethyl group, 1-phenylpropyl group, 1-phenylbutyl group, 2-methyl-1-phenylpropyl group, 1-phenylpentyl group, 2-methyl-1-phenylbutyl group, 3-methyl-1-phenylbutyl group, diphenylmethyl group, 1,1-diphenylethyl group, triphenylmethyl group, naphthylmethyl group and 1-naphthylethyl group; alkyl ether type protecting groups such as methyl group, tert-butyl group, 1-ethoxyethyl group, 3,4,5,6-tetrahydro-2H-pyran-2-yl group, triphenylmethyl group, 1-methoxy-1-methylethyl group, methoxymethyl group and 2-methoxyethoxymethyl group; silyl type protecting groups such as trimethylsilyl group, triethylsilyl group, tripropylsilyl group, triisopropylsilyl group, tributylsilyl group, tert-butyldimethylsilyl group and tert-butyldiphenylsilyl group; and ester type protecting groups such as acetyl group, propanoyl group, butanoyl group, isobutanoyl group, pivaloyl group, benzoyl group, 4-nitrobenzoyl group, 4-methoxybenzoyl group, 4-methylbenzoyl group, 4-tert-butylbenzoyl group, 4-fluorobenzoyl group, 4-chlorobenzoyl group, 4-bromobenzoyl group, 3-nitrobenzoyl group, 3-methoxybenzoyl group, 3-methylbenzoyl group, 3-tert-butylbenzoyl group, 3-fluorobenzoyl group, 3-chlorobenzoyl group, 3-bromobenzoyl group, 2-nitrobenzoyl group, 2-methoxybenzoyl group, 2-methylbenzoyl group, 2-tert-butylbenzoyl group, 2-fluorobenzoyl group, 2-chlorobenzoyl group, 2-bromobenzoyl group, 3,5-dinitrobenzoyl group, 3,5-dimethoxybenzoyl group, 3,5-dimethylbenzoyl group, 3,5-di-tert-butylbenzoyl group, 3,5-difluorobenzoyl group, 3,5-dichlorobenzoyl group, 3,5-dibromobenzoyl group, 2,4-dinitrobenzoyl group, 2,4-dimethoxybenzoyl group, 2,4-dimethylbenzoyl group, 2,4-di-tert-butylbenzoyl group, 2,4-difluorobenzoyl group, 2,4-dichlorobenzoyl group, 2,4-dibromobenzoyl group, 2,5-dinitrobenzoyl group, 2,5-dimethoxybenzoyl group, 2,5-dimethylbenzoyl group, 2,5-di-tert-butylbenzoyl group, 2,5-difluorobenzoyl group, 2,5-dichlorobenzoyl group, 2,5-dibromobenzoyl group, 4-phenylbenzoyl group, 2-phenylbenzoyl group, 4-methoxycarbonylbenzoyl group, 3-methoxycarbonylbenzoyl group and 2-methoxycarbonylbenzoyl group; and among them, benzyl ether type protecting groups are preferred, and benzyl group is particularly preferred.

The C₁ to C₆ alkyl group represented by R³ may be either linear or branched, and examples thereof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

Next, each step will be described in more detail.

### (Step A)

For the sulfonyl halide represented by formula (1) and the alcohol represented by formula (11) in Step A, commercially available products can be used as they are, and they can also be prepared by a known method.

The present step can also be carried out without solvent, but is preferably carried out in a solvent in that the reaction mixture easily flows and it is good in handling.

Examples of the solvent used in the step include aromatic hydrocarbon solvents such as benzene, toluene, xylene and chlorobenzene; aliphatic hydrocarbon solvents such as hexane, pentane and cyclohexane; ether solvents such as diethyl ether, diisopropyl ether, tert-butyl methyl ether, tetrahydrofuran, 1,4-dioxane and 1,2-dimethoxyethane; halogenated hydrocarbon solvents such as dichloromethane, chloroform, 1,2-dichloroethane and carbon tetrachloride; nitrile solvents such as acetonitrile and propionitrile; aprotic polar solvents such as N,N-dimethylformamide, N,N-dimethylacetamide, N-methylpyrrolidone and dimethyl sulfoxide; ester solvents such as methyl acetate, ethyl acetate, butyl acetate and tert-butyl acetate; and ketone solvents such as acetone and methyl ethyl ketone. Among them, aromatic hydrocarbon solvents are preferred, and toluene is particularly preferred, in that the post-treatment is easier.

The amount of the solvent used is preferably a ratio of 0.3 to 10 mL and more preferably 0.4 to 2.0 mL based on 1 mmol of the sulfonyl halide represented by formula (1).

The amount of the alcohol represented by formula (11) used is preferably a ratio of 0.8 to 2.0 mol and more preferably 0. 9 to 1.1 mol based on 1 mol of the sulfonyl halide represented by formula (1).

Examples of the base used in the present step include inorganic bases such as sodium hydroxide, potassium hydroxide, lithium hydroxide, barium hydroxide, sodium carbonate, potassium carbonate, sodium bicarbonate and potassium bicarbonate; and organic bases, for example, amine compounds such as triethylamine, diisopropylethylamine, N-methylmorpholine, 1,8-diazabicyclo[5.4.0]undec-7-ene (DBU), 1,5-diazabicyclo[4.3.0]non-5-ene (DBN), pyridine and 2,6-lutidine. Among them, weak bases, particularly amine compounds are preferred, and triethylamine is particularly preferred, in that a side reaction can be well suppressed.

The amount of the base used is preferably a ratio of 0.8 to 2.0 mol and more preferably 0.9 to 1.1 mol based on 1 mol of the sulfonyl halide represented by formula (1).

The present step is carried out by a method of adding the sulfoyl halide represented by formula (1) to a mixture of the alcohol represented by formula (11), the base and the solvent; a method of adding the base to a mixture of the alcohol, the sulfonyl halide and the solvent; a method of adding the alcohol to a mixture of the sulfonyl halide, the base and the solvent; or the like, and a method of adding the sulfonyl halide to a mixture of the alcohol, the base and the solvent is preferred in that the reactivity of the sulfonyl halide is better.

The reaction temperature is preferably within a range from 0 to 40°C and more preferably 0 to 10°C.

The reaction time is usually 1 to 10 hours and preferably 1 to 3 hours, while it also depends on the types of the sulfonyl halide represented by formula (1), the alcohol represented by formula (11) and the base, and the reaction temperature.

The sulfonic acid ester represented by formula (2) thus obtained can be isolated and purified by an ordinary method. For example, the sulfonic acid ester can be isolated and purified after performing an extraction operation or by subjecting the reaction mixture directly to silica gel column chromatography. In addition, the reaction mixture can also be subjected to the next step without isolation or purification.

### (Step B)

The sulfonic acid ester represented by formula (4) can be produced by reacting the sulfonic acid ester represented by formula (2) with the epoxy compound represented by formula (31) in the presence of a base.

The present step can also be carried out without solvent, but is preferably carried out in a solvent in that the reaction mixture easily flows and it is good in handling.

Examples of the solvent used in the step include aromatic hydrocarbon solvents such as benzene, toluene, xylene and chlorobenzene; aliphatic hydrocarbon solvents such as hexane, pentane and cyclohexane; ether solvents such as diethyl ether, diisopropyl ether, tert-butyl methyl ether, tetrahydrofuran, 1,4-dioxane and 1,2-dimethoxyethane; and halogenated hydrocarbon solvents such as dichloromethane, chloroform, 1, 2-dichloroethane and carbon tetrachloride. Among them, when the base is an alkylalkali metal, ether solvents, aromatic hydrocarbon solvents and aliphatic hydrocarbon solvents are preferred, and tetrahydrofuran, toluene and hexane are particularly preferred, in that the alkylalkali metal is not remarkably decomposed.

The amount of the solvent used is preferably a ratio of 0.3 to 10 mL and more preferably 0.5 to 2.0 mL based on 1 mmol of the sulfonic acid ester represented by formula (2).

The amount of the epoxy compound represented by formula (31) used is preferably a ratio of 0.8 to 2.0 mol and more preferably 0.9 to 1.1 mol based on 1 mol of the sulfonic acid ester represented by formula (2).

Examples of the base used in the present step include alkali metal hydrides such as lithium hydride, sodium hydride and potassium hydride; alkali metal amides such as lithium amide, sodium amide and lithium diisopropylamide; alkylalkali metals such as n-butyllithium, sec-butyllithium and tert-butyllithium; and alkali metal alkoxides such as sodium methoxide, sodium ethoxide, potassium tert-butoxide and sodium tert-butoxide. Among them, strong bases, particularly, alkylalkali metals are preferred, alkyllithium is more preferred, and n-butyllithium is particularly preferred, from the viewpoint of having good reactivity. n-Butyllithium is usually used in the form of a hexane solution.

The amount of the base used is preferably a ratio of 1.8 to 3.0 equivalents and more preferably 2.0 to 2.2 equivalents based on 1 equivalent of the sulfonic acid ester represented by formula (2).

The present step is carried out by a method of adding the base to a mixture of the sulfonic acid ester represented by formula (2), the epoxy compound represented by formula (31) and the solvent; a method of adding the epoxy compound to a mixture of the sulfonic acid ester, the base and the solvent; a method of adding the sulfonic acid ester to a mixture of the epoxy compound, the base and the solvent; or the like, and a method of adding the base to a mixture of the sulfonic acid ester, the epoxy compound and the solvent is preferred in that a side reaction can be well suppressed.

The reaction temperature is preferably within a range from -80 to 0°C and more preferably -40 to -20°C.

The reaction time is usually 0.5 to 15 hours and preferably 1 to 5 hours, while it also depends on the types of the sulfonic acid ester represented by formula (2), the epoxy compound represented by formula (31) and the base, and the reaction temperature.

The sulfonic acid ester represented by formula (4) thus obtained can be isolated and purified by an ordinary method. For example, the sulfonic acid ester compound (4) can be isolated and purified after performing an extraction operation or by subjecting the reaction mixture directly to silica gel column chromatography. Alternatively, the reaction mixture can also be subjected to the next step without isolation and purification.

The sulfonic acid ester represented by formula (4) can also be produced by a method through a step of reacting the sulfonic acid ester represented by formula (2) with a base to obtain the cyclopropanesulfonic acid ester represented by formula (3) (Step B'-1) and a step of reacting the cyclopropanesulfonic acid ester represented by formula (3) with the epoxy compound represented by formula (31) in the presence of a base (Step B'-2).

### (Step B'-1)

The present step is a step of reacting the sulfonic acid ester represented by formula (2) with a base to obtain the cyclopropanesulfonic acid ester represented by formula (3).

Examples of the base used in the present step include alkali metal hydrides such as lithium hydride, sodium hydride and potassium hydride; alkali metal amides such as lithium amide, sodium amide and lithium diisopropylamide; alkylalkali metals such as n-butyllithium, sec-butyllithium and tert-butyllithium; and alkali metal alkoxides such as sodium methoxide, sodium ethoxide, potassium tert-butoxide and sodium tert-butoxide. Among them, strong bases, particularly, alkylalkali metals are preferred, alkyllithium is more preferred, and n-butyllithium is particularly preferred, from the viewpoint of having good reactivity. n-Butyllithium is usually used in the form of a hexane solution.

The amount of the base used is preferably a ratio of 0.9 to 1.5 equivalents and more preferably 1.0 to 1.2 equivalents based on 1 equivalent of the sulfonic acid ester represented by formula (2).

The present step can also be carried out without solvent, but is preferably carried out in a solvent in that the reaction mixture easily flows and it is good in handling.

Examples of the solvent used in the step include aromatic hydrocarbon solvents such as benzene, toluene, xylene and chlorobenzene; aliphatic hydrocarbon solvents such as hexane, pentane and cyclohexane; ether solvents such as diethyl ether, diisopropyl ether, tert-butyl methyl ether, tetrahydrofuran, 1,4-dioxane and 1,2-dimethoxyethane; and halogenated hydrocarbon solvents such as dichloromethane, chloroform, 1, 2-dichloroethane and carbon tetrachloride. Among them, when the base is a preferable alkylalkali metal, ether solvents and aliphatic hydrocarbon solvents are preferred, and tetrahydrofuran and hexane are particularly preferred, in that the alkylalkali metal is not remarkably decomposed.

The amount of the solvent used is preferably a ratio of 0.3 to 10 mL and more preferably 0.5 to 2.0 mL based on 1 mmol of the sulfonic acid ester compound (2).

The present step is carried out by a method of adding the base to a mixture of the sulfonic acid ester represented by formula (2) and the solvent; a method of adding the sulfonic acid ester to a mixture of the base and the solvent; or the like, and a method of adding the base to a mixture of the sulfonic acid ester and the solvent is preferred in that a side reaction can be well suppressed.

The reaction temperature is preferably within a range from -80 to 0°C and more preferably -80 to -20°C.

The reaction time is usually 0.5 to 15 hours and preferably 1 to 5 hours, while it also depends on the types of the sulfonic acid ester represented by formula (2) and the base, and the reaction temperature.

The cyclopropanesulfonic acid ester represented by formula (3) thus obtained can be isolated and purified by an ordinary method. For example, the cyclopropanesulfonic acid ester represented by formula (3) can be isolated and purified after performing an extraction operation or by subjecting the reaction mixture directly to silica gel column chromatography. Alternatively, the reaction mixture can also be subjected to the following step B'-2 without isolation or purification.

### (Step B'-2)

The present step is a step of reacting the cyclopropanesulfonic acid ester represented by formula (3) with the epoxy compound represented by formula (31) in the presence of a base to obtain the sulfonic acid ester represented by formula (4).

Examples of the base used in the present step include alkali metal hydrides such as lithium hydride, sodium hydride and potassium hydride; alkali metal amides such as lithium amide, sodium amide and lithium diisopropylamide; alkylalkali metals such as n-butyllithium, sec-butyllithium and tert-butyllithium; and alkali metal alkoxides such as sodium methoxide, sodium ethoxide, potassium tert-butoxide and sodium tert-butoxide. Among them, strong bases, particularly, alkylalkali metals are preferred, alkyllithium is more preferred, and n-butyllithium is particularly preferred, from the viewpoint of having good reactivity. n-Butyllithium is usually used in the form of a hexane solution.

The amount of the base used is preferably a ratio of 0.9 to 1.5 equivalents and more preferably 1.0 to 1.2 equivalents based on 1 equivalent of the cyclopropanesulfonic acid ester represented by formula (3).

The present step can also be carried out without solvent, but is preferably carried out in a solvent in that the reaction mixture easily flows and it is good in handling.

Examples of the solvent used in the step include aromatic hydrocarbon solvents such as benzene, toluene, xylene and chlorobenzene; aliphatic hydrocarbon solvents such as hexane, pentane and cyclohexane; ether solvents such as diethyl ether, diisopropyl ether, tert-butyl methyl ether, tetrahydrofuran, 1,4-dioxane and 1,2-dimethoxyethane; and halogenated hydrocarbon solvents such as dichloromethane, chloroform, 1, 2-dichloroethane and carbon tetrachloride. Among them, when the base is a preferable alkylalkali metal, ether solvents, aromatic hydrocarbon solvents and aliphatic hydrocarbon solvents are preferred, and tetrahydrofuran, toluene and hexane are particularly preferred, in that the alkylalkali metal is not remarkably decomposed.

The amount of the solvent used is preferably a ratio of 0.3 to 10 mL and more preferably 0.5 to 2.0 mL based on 1 mmol of the cyclopropanesulfonic acid ester represented by formula (3).

The amount of the epoxy compound represented by formula (31) used is preferably a ratio of 0.8 to 2.0 mol and more preferably 0.9 to 1.1 mol based on 1 mol of the cyclopropanesulfonic acid ester represented by formula (3).

The present step is carried out by a method of adding the epoxy compound represented by formula (31) to a mixture of the cyclopropanesulfonic acid ester represented by formula (3), the base and the solvent; a method of adding the cyclopropanesulfonic acid ester to a mixture of the epoxy compound, the base and the solvent; a method of adding the base to a mixture of the cyclopropanesulfonic acid ester, the epoxy compound and the solvent; or the like, and a method of adding the base to a mixture of the cyclopropanesulfonic acid ester, the epoxy compound and the solvent is preferred in that a side reaction can be well suppressed.

The reaction temperature is preferably within a range from -80 to 0°C and more preferably -80 to -20°C.

The reaction time is usually 0.5 to 15 hours and preferably 1 to 5 hours, while it also depends on the types of the cyclopropanesulfonic acid ester represented by formula (3), the epoxy compound represented by formula (31) and the base, and the reaction temperature.

The sulfonic acid ester represented by formula (4) thus obtained can be isolated and purified by an ordinary method. For example, the sulfonic acid ester represented by formula (4) can be isolated and purified after performing an extraction operation or by subjecting the reaction mixture directly to silica gel column chromatography. Alternatively, the reaction mixture can also be subjected to the next step without isolation or purification.

Configuration of the carbon atom to which * is attached in the sulfonic acid ester represented by formula (4) may be either the S configuration or the R configuration.

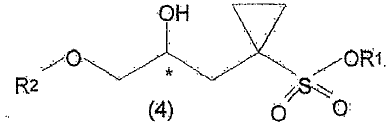

In order to obtain an optically active sulfonic acid ester represented by formula (4), depending on the desired configuration of the carbon atom to which * is attached in the above formula, one having a configuration of the carbon atom to which * is attached in the epoxy compound (31) represented by formula (31) may be selected as a raw material.

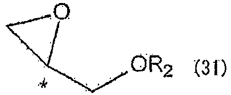

### (Step C)

The sulfonic acid ester represented by formula (5) can be produced by deprotecting the sulfonic acid ester represented by formula (4).

The method of deprotecting the sulfonic acid ester represented by formula (5) is selected depending on the protecting group of a hydroxyl group of R², and when R² is a benzyl ether protecting group, deprotection can be performed by catalytic reduction. Catalytic reduction is performed by reacting the sulfonic acid ester represented by formula (4) in a solvent in the presence of a metal catalyst and a hydrogen source.

Examples of the metal catalyst used include palladium-carbon, palladium black, palladium chloride, palladium hydroxide, rhodium-carbon, platinum oxide, platinum black, platinum-palladium, Raney nickel, and Raney cobalt. Among them, palladium-carbon is preferred from the viewpoint of reactivity of the sulfonic acid ester represented by formula (4).

The amount of the metal catalyst used is usually a ratio of 0.01 to 0. 30 times by weight and preferably 0. 03 to 0.10 times by weight based on that of the sulfonic acid ester represented by formula (4).

Examples of the hydrogen source used include hydrogen gas.

The present step can also be carried out without solvent, but is preferably carried out in a solvent in that the reaction mixture easily flows and it is good in handling.

Examples of the solvent used in the present step include alcohol solvents such as methanol, ethanol, propanol, 2-propanol, butanol, isobutanol, tert-butanol and cyclohexanol; ether solvents such as diethyl ether, diisopropyl ether, tert-butyl methyl ether, tetrahydrofuran, 1,4-dioxane and 1,2-dimethoxyethane; aromatic hydrocarbon solvents such as benzene, toluene, xylene and chlorobenzene; aliphatic hydrocarbon solvents such as hexane, pentane and cyclohexane; and halogenated hydrocarbon solvents such as dichloromethane, chloroform, 1,2-dichloroethane and carbon tetrachloride. Among them, alcohol solvents are preferred, and ethanol is particularly preferred, from the viewpoint of reactivity.

The amount of the solvent used is preferably a ratio of 0.3 to 10 mL and more preferably 0.5 to 2.0 mL based on 1 mmol of the sulfonic acid ester represented by formula (4).

The present step is carried out by a method of adding the hydrogen source to a mixture of the sulfonic acid ester represented by formula (4), the metal catalyst and the solvent.

The reaction temperature is preferably within a range from 10 to 50°C and more preferably 20 to 30°C.

The reaction time is, for example, 1 to 10 hours and preferably 3 to 5 hours, while it also depends on the type of the sulfonic acid ester represented by formula (4) and the reaction temperature.

The sulfonic acid ester represented by formula (5) thus obtained can be isolated and purified by an ordinary method. For example, the sulfonic acid ester compound (5) can be isolated and purified after performing an extraction operation or by subjecting the reaction mixture directly to silica gel column chromatography. Alternatively, the reaction mixture can also be subjected to the next step without isolation or purification.

Configuration of the carbon atom to which * is attached in the sulfonic acid ester compound (5) may be either the S configuration or the R configuration.

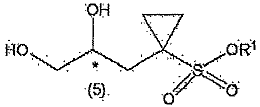

In order to obtain an optically active sulfonic acid ester represented by formula (5), depending on the desired configuration of the carbon atom to which * is attached in the above formula, one having a configuration of the carbon atom to which * is attached in the sulfonic acid ester represented by formula (4) may be selected as a raw material.

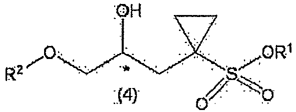

### (Step D)

The sulfonic acid ester represented by formula (6) can be produced by reacting the sulfonic acid ester represented by formula (5) with the2,2-dialkoxypropane represented by formula (51) in the presence of an acid.

The present step can also be carried out without solvent, but is preferably carried out in a solvent in that the reaction mixture easily flows and it is good in handling.

Examples of the solvent used in the step include ketone solvents such as acetone and methyl ethyl ketone; aromatic hydrocarbon solvents such as benzene, toluene, xylene and chlorobenzene; aliphatic hydrocarbon solvents such as hexane, pentane and cyclohexane; halogenated hydrocarbon solvents such as dichloromethane, chloroform, 1,2-dichloroethane and carbon tetrachloride; and ester solvents such as methyl acetate, ethyl acetate, butyl acetate and tert-butyl acetate. Among them, ketone solvents are preferred, and acetone is particularly preferred, from the viewpoint of reactivity.

The amount of the solvent used is preferably a ratio of 0.5 to 10 mL and more preferably 1 to 5 mL based on 1 mmol of the sulfonic acid ester represented by formula (5).

The amount of the 2,2-dialkoxypropane represented by formula (51) used is usually a ratio of 1.0 to 2.0 mol and preferably 1.3 to 1.7 mol based on 1 mol of the sulfonic acid ester represented by formula (5).

Examples of the acid used in the present step include hydrochloric acid, sulfuric acid, nitric acid and acetic acid. Among them, sulfuric acid is preferred because of excellence in reactivity of the sulfonic acid ester represented by formula (5). The amount of the acid used is usually a ratio of 0.0001 to 0.01 equivalents and preferably 0.0005 to 0.002 equivalents based on 1 equivalent of the sulfonic acid ester represented by formula (5).

The present step is carried out by a method of adding the acid to a mixture of the sulfonic acid ester represented by formula (5), the 2,2-dialkoxypropane represented by formula (51) and the solvent; a method of adding the 2, 2-dialkoxypropane to a mixture of the sulfonic acid ester, the acid and the solvent; a method of adding the sulfonic acid ester to a mixture of the 2,2-dialkoxypropane, the acid and the solvent; or the like, and a method of adding the acid to a mixture of the sulfonic acid ester, the 2, 2-dialkoxypropane and the solvent is preferred in that a side reaction can be well suppressed.

The reaction temperature is preferably within a range from 0 to 50°C and more preferably 20 to 30°C.

The reaction time is usually 1 to 20 hours and preferably 2 to 10 hours, while it also depends on the types of the sulfonic acid ester represented by formula (5), the 2,2-dialkoxypropane represented by formula (51) and the acid, and the reaction temperature.

The sulfonic acid ester represented by formula (6) thus obtained can be isolated and purified by an ordinary method. For example, the sulfonic acid ester represented by formula (6) can be isolated and purified after performing an extraction operation or by subjecting the reaction mixture directly to silica gel column chromatography. Alternatively, the reaction mixture can also be subj ected to the next step without isolation or purification.

Configuration of the carbon atom to which * is attached in the sulfonic acid ester represented by formula (6) may be either the S configuration or the R configuration.

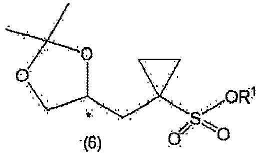

In order to obtain an optically active sulfonic acid ester represented by formula (6), depending on the desired configuration of the carbon atom to which * is attached in the above formula, one having a configuration of the carbon atom to which * is attached in the sulfonic acid ester represented by formula (5) may be selected as a raw material.

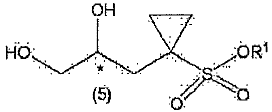

### (Step E)

The sulfonate represented by formula (7) can be produced by reacting the sulfonic acid ester represented by formula (6) with sodium iodide.

The present step can also be carried out without solvent, but is preferably carried out in a solvent in that the reaction mixture easily flows and it is good in handling.

Examples of the solvent used include ketone solvents such as acetone and methyl ethyl ketone, and water. Among them, ketone solvents are preferred, and acetone is particularly preferred, from the viewpoint of reactivity.

The amount of the solvent used is preferably a ratio of 0.5 to 10 mL and more preferably 1 to 5 mL based on 1 mmol of the sulfonic acid ester represented by formula (6).

The amount of sodium iodide used is usually a ratio of 1.0 to 2.0 mol and preferably 1.1 to 1.7 mol based on 1 mol of the sulfonic acid ester represented by formula (6).

The present step is carried out by a method of adding sodium iodide to a mixture of the sulfonic acid ester represented by formula (6) and the solvent; a method of adding the sulfonic acid ester to a mixture of sodium iodide and the solvent; or the like, and a method of adding sodium iodide to a mixture of the sulfonic acid ester and the solvent is preferred in that a side reaction can be well suppressed.

The reaction temperature is preferably within a range from 30 to 55°C and more preferably 45 to 55°C.

The reaction time is usually 20 to 40 hours and preferably 20 to 30 hours, while it also depends on the type of the sulfonic acid ester represented by formula (6) and the reaction temperature.

The sulfonate represented by formula (7) can also be produced by reacting the sulfonic acid ester represented by formula (6) with sodium hydroxide.

The present step can also be carried out without solvent, but is preferably carried out in a solvent in that the reaction mixture easily flows and it is good in handling.

Examples of the solvent used in the step include ketone solvents such as acetone and methyl ethyl ketone; and water. Among them, ketone solvents are preferred, and acetone is particularly preferred, from the viewpoint of reactivity.

The amount of the solvent used is preferably a ratio of 0.5 to 10 mL and more preferably 1 to 5 mL based on 1 mmol of the sulfonic acid ester represented by formula (6).

The amount of sodium hydroxide used is usually a ratio of 1.0 to 2.0 mol and preferably 1.1 to 1.7 mol based on 1 mol of the sulfonic acid ester represented by formula (6).

The present step is carried out by a method of adding sodium hydroxide to a mixture of the sulfonic acid ester represented by formula (6) and the solvent; a method of adding the sulfonic acid ester to a mixture of sodium hydroxide and the solvent; or the like, and a method of adding sodium hydroxide to a mixture of the sulfonic acid ester and the solvent is preferred in that a side reaction can be well suppressed.

The reaction temperature is preferably within a range from 0 to 55°C and more preferably 0 to 30°C.

The reaction time is usually 1 to 10 hours and preferably 1 to 5 hours, while it also depends on the type of the sulfonic acid ester represented by formula (6) and the reaction temperature.

The sulfonate represented by formula (7) thus obtained can be isolated and purified by an ordinary method. For example, the sulfonate can be isolated and purified after performing an extraction operation or by subjecting the reaction mixture directly to silica gel column chromatography.

Configuration of the carbon atom to which * is attached in the sulfonate represented by formula (7) may be either the S configuration or the R configuration.

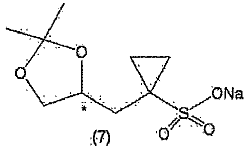

In order to obtain an optically active sulfonate represented by formula (7), depending on the desired configuration of the carbon atom to which * is attached in the above formula, one having a configuration of the carbon atom to which * is attached in the sulfonic acid ester represented by formula (6) may be selected as a raw material.

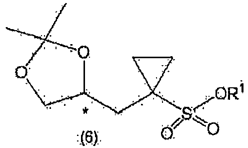

### EXAMPLES

Hereinafter, the present invention will be described in further detail by Examples.

### Example 1: Production of Compound Represented by Formula (2A)

### (Step A)

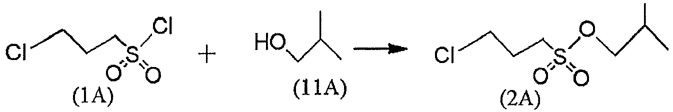

Under a nitrogen gas atmosphere, 242.2 g of toluene, 30.8 g of isobutyl alcohol (1.05 times by mol to 3-chloropropanesulfanyl chloride) and 41.2 g of triethylamine (1.03 times by mol to 3-chloropropanesulfonyl chloride) were mixed at room temperature, and the resulting mixture was cooled to 3°C. Thereto was added dropwise 70 g of 3-chloropropanesulfonyl chloride while keeping the temperature of the resulting mixture in a range from 3°C to 8°C. About 3 hours were taken for the dropwise addition. After completion of the dropwise addition, 30.3 g of toluene was added to the resulting mixture, and the resulting mixture was stirred at a temperature ranging from 2°C to 5°C for 1 hour. After completion of the reaction, 140 g of water was added dropwise to the resulting mixture while keeping the temperature of the resulting mixture in a range from 0°C to 10°C, and 2.1 g of 35% by weight of hydrochloric acid was further added dropwise. The temperature of the resulting mixture was adj usted within a range from 20°C to 25°C, the resulting mixture was stirred at that temperature for 30 minutes, and then an organic layer obtained by separation was washed with an aqueous sodium bicarbonate solution. The separated organic layer was concentrated under reduced pressure to obtain 90 g of a compound represented by formula (2A). The chemical purity of the compound represented by formula (2A) was 95.1%, and the yield was 100%.
¹H-NMR (400MHz,CDCl₃)δ: 4.02(2H,d), 3.70(2H,t), 3.30(2H,t), 2.34(2H,m), 2.05(1H,m), 0.99(6H,d)

### Example 2: Production of Compound Represented by Formula (2)

### (Step A)

The same experiment as in Example 1 is carried out except for replacing isobutyl alcohol in Example 1 with neopentyl alcohol, and thus a compound represented by formula (2) wherein X¹ is a chlorine atom and R¹ is a neopentyl group can be produced. Example 3: Production of Compound Represented by Formula (3A)

### (Step B'-1)

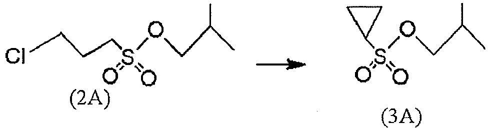

The compound represented by formula (2A) obtained in Example 1 (130 g) and 920 mL of tetrahydrofuran were mixed at room temperature, and the mixture was cooled to -72°C. A 2.5 mol/L n-butyllithium/hexane solution (the amount of n-butyllithium was 1.1 times by mol that of the compound represented by formula (2A)) was added dropwise thereto at a temperature ranging from -72°C to -67°C over 1 hour. After the dropwise addition, the resulting mixture was stirred at a temperature ranging from -77°C to -72°C for 3 hours, and the resulting mixture was heated to around 0°C. Thereto were added 130 mL of water, 650 mL of methyl tert-butyl ether and 130 mL of aqueous 5% by weight acetic acid, and the mixture was stirred at a temperature ranging from -18°C to -11°C for 30 minutes. After separation, the resulting aqueous layer was mixed with 390 mL of methyl tert-butyl ether, and the organic layer obtained by separation was combined with the organic layer obtained by the previous separation. The combined organic layers were concentrated under reduced pressure to obtain 103 g of a compound represented by formula (3A). The chemical purity of the compound represented by formula (3A) was 96.7%, and the yield was 93%.
(¹H-NMR(400MHz),CDCl₃)δ: 4.01(2H,d), 2.47(1H,m), 2.04(1H,m), 1.27(2H,m), 1.07(2H,m), 0.99(6H,d)

### Example 4: Production of Compound Represented by Formula (3)

The same experiment as in Examples 1 and 3 is carried out except for replacing isobutyl alcohol in Example 1 with neopentyl alcohol, and thus a compound represented by formula (3) wherein R¹ is a neopentyl group can be produced. Example 5: Production of Compound Represented by Formula (4A)

### (Step B)

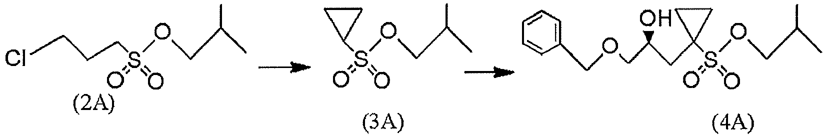

Under a nitrogen gas atmosphere, 42.4 g of the compound represented by formula (2A) obtained in Example 1, 71.3 g of tetrahydrofuran, 30.8 g of (S)-benzyl glycidyl ether (1 time by mol that of the compound represented by formula (2A)) and 69.7 g of toluene were mixed at room temperature, and the resulting mixture was cooled to -30°C. Thereto was added dropwise 167 g of a 15% by weight n-butyllithium/hexane solution (the amount of n-butyllithium was 2.1 times by mol that of the compound represented by formula (2A)) at a temperature ranging from -30°C to -25°C over about 6 hours. After completion of the dropwise addition, the resulting reaction mixture was kept at a temperature ranging from -30°C to -25°C for about 1.5 hours. The resulting reaction mixture was added dropwise to aqueous acetic acid prepared by diluting 14.7 g of acetic acid with 141 g of water, at a temperature ranging from 3°C to 10°C over 20 minutes. To the resulting mixture was added 52 g of toluene, the mixture was stirred at a temperature ranging from 20°C to 25°C for 30 minutes, and an organic layer obtained by separation was washed with an aqueous sodium bicarbonate solution, followed by water. The resulting organic layer was concentrated under reduced pressure to obtain a compound represented by formula (4A). The chemical purity of the compound represented by formula (4A) was 83%, and the yield was 87%.
¹H-NMR(400MHz,CDCl₃)δ: 7.30-7.37(5H,m), 4.55(2H,d), 4.20(1H,m), 4.00(2H,m), 3.51(1H,dd), 3.41(1H,dd), 2.61(1H,d), 2.13(1H,dd), 2.03(1H,m), 1.83(1H,dd), 1.45(2H,m), 1.20(1H,m), 0.98(6H,d), 0.96(1H,m)

### Example 6: Production of Compound Represented by Formula (4)

The same experiment as in Examples 1, 3 and 5 is carried out except for replacing isobutyl alcohol in Example 1 with neopentyl alcohol, and thus a compound represented by formula (4) wherein R¹ is a neopentyl group and R² is a benzyl group can be produced.

### Example 7: Production of Compound Represented by Formula (5A)

### (Step C)

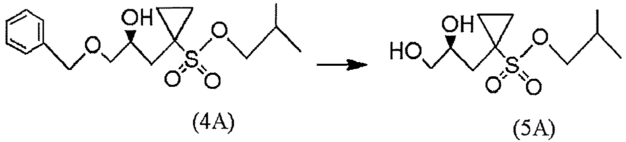

The compound represented by formula (4A) obtained in Example 5 (66.5 g), 26.3 g of ethanol and 86.4 g of cyclohexane were mixed at about 20°C, and a mixture obtained by suspending 2.78 g of activated carbon in 17.5 g of ethanol was added to the resulting mixture at about 25°C. The resulting mixture was stirred at about 25°C for 1 hour and then filtered. A solid content removed by filtration was washed with a mixed liquid of 11 g of ethanol and 22 g of cyclohexane, and the obtained washing liquid and the previously obtained filtrate were mixed. The resulting mixed liquid was adjusted to be at around 20°C in an autoclave under a nitrogen gas atmosphere, and 5.55 g of 10% by weight palladium carbon was added thereto. After sealing the autoclave, the inside of the autoclave was pressurized with hydrogen gas to be at 0. 2 MPa to 0. 3 MPa. The mixture was stirred under a pressure of hydrogen gas of 0.2 MPa to 0.3 MPa at a temperature ranging from 23°C to 26°C for about 3.5 hours, and then the gas phase part in the autoclave was replaced with nitrogen gas. The resulting reaction mixture was filtered, and the solid content removed by filtration was washed with a mixed liquid of 11 g of ethanol and 22 g of cyclohexane, and.the obtained washing liquid and the previously obtained filtrate were mixed. Water and toluene were added to the resulting mixed liquid, the mixture was separated, and then the resulting aqueous layer was extracted with a mixed liquid of toluene and cyclohexane. A common salt was added to the resulting aqueous layer, and the mixture was further extracted twice with ethyl acetate. The organic layers extracted with ethyl acetate were combined and then concentrated under reduced pressure to obtain a compound represented by formula (5A). The chemical purity of the compound represented by formula (5A) was 97%, and the yield was 99%.
¹H-NMR(400MHz, CDCl₃)δ: 4.12(1H,m), 4.02(2H,m), 3.66(1H,dd), 3.47(1H,dd), 3.15(1H,br s), 2.44(1H,br s), 2.03(2H,m), 1.86(1H,dd), 1.49(2H,m), 1.15(1H,m), 1.00(6H,d), 0.96(1H,m) Example 8: Production of Compound Represented by Formula (5)

The same experiment as in Examples 1, 3, 5 and 7 is carried out except for replacing isobutyl alcohol in Example 1 with neopentyl alcohol, and thus a compound represented by formula (5) wherein R¹ is a neopentyl group can be produced.

### Example 9: Production of Compound Represented by Formula (6A)

### (Step D)

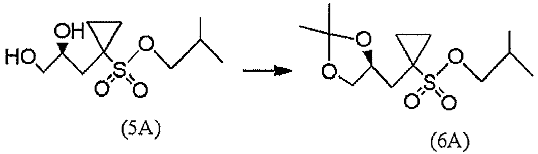

The compound represented by formula (5A) obtained in Example 7 (83.8 g), 642 g of acetone and 50.3 g (1.5 mol based on 1 mol of the compound represented by formula (5A)) of 2,2-dimethoxypropane were mixed, and the resulting mixture was adjusted to be at about 25°C. Thereto was added dropwise 31.6 mg (0.001 mol based on 1 mol of the compound represented by formula (5A)) of concentrated sulfuric acid, and the resulting mixture was stirred at 24°C to 28°C for 8 hours to obtain a compound represented by formula (6A).
¹H-NMR(400MHz,CDCl₃)δ: 4.41(1H,m), 4.12(1H,dd), 4.00(2H,m), 3.47(1H,dd), 2.35(1H,dd), 2.04(1H,m), 1.83(1H,dd), 1.45(2H,m), 1.38(3H,s), 1.34(3H,s), 1.23(1H,m), 0.98(7H,m)

### Example 10: Production of Compound Represented by Formula (6)

The same experiment as in Examples 1, 3, 5, 7 and 9 is carried out except for replacing isobutyl alcohol in Example 1 with neopentyl alcohol, and thus a compound represented by formula (6) wherein R¹ is a neopentyl group can be produced. Example 11: Production of Compound Represented by Formula (7A)

### (Step E)

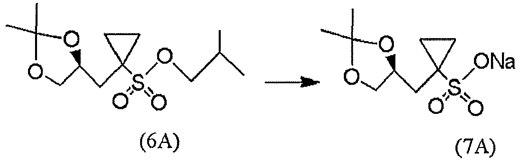

The compound represented by formula (6A) obtained in Example 9 (776 g) (about 12% by weight acetone solution) and 65.2 mg of triethylamine (0.002 times by mol to the compound represented by formula (6A)) were mixed at 25°C, and 72.4 g of sodium iodide (1.5 times by mol to the compound represented by formula (6A)) was added to the resulting mixture at a
temperature ranging from 25°C to 30°C. The resulting mixture was adjusted to be at about 50°C, and stirred for 25 hours. The resulting reaction mixture was cooled to around 20°C, and triethylamine was added thereto to adjust the pH of the mixture to 10. The mixture of which pH was adjusted was stirred at 20°C for 10 minutes and then filtered, and the resulting solid was washed with acetone. The solid washed was concentrated under reduced pressure to obtain a compound represented by formula (7A). The yield was 77%.
¹H-NMR(400MHz, DMSO-d₆)δ: 4.43(1H,m), 4.01(1H, dd), 3.47(1H,dd), 1.96(1H,dd), 1.69(1H,dd), 1.25(3H,s), 1.21(3H,s), 0.84(2H,dd), 0.44(2H,m)

### Example 12: Production of Compound Represented by Formula (7)

The same experiment as in Examples 1, 3, 5, 7, 9 and 11 is carried out except for replacing isobutyl alcohol in Example 1 with neopentyl alcohol, and thus a compound represented by formula (7) can be produced.

### INDUSTRIAL APPLICATBILITY

The compound represented by formula (7) is a compound useful as a precursor for a chemotherapeutic medicament. The present invention is industrially applicable as a novel compound that can be used in producing the compound represented by formula (7) and a production method thereof.

## Claims

1. A method for producing a sulfonate represented by formula (7) : 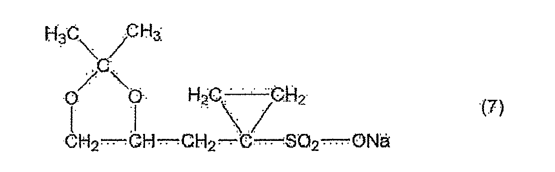 which comprises:
(Step E)
a step of reacting a sulfonic acid ester represented by formula (6): 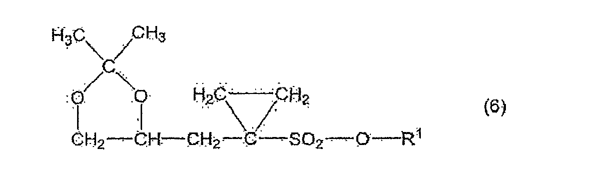 wherein R¹ represents a C₄ to C₆ branched alkyl group,
with sodium iodide or sodium hydroxide to obtain the sulfonate represented by formula (7).

2. The method for producing the sulfonate according to claim 1, which comprises:
(Step D)
a step of reacting a sulfonic acid ester represented by formula (5): 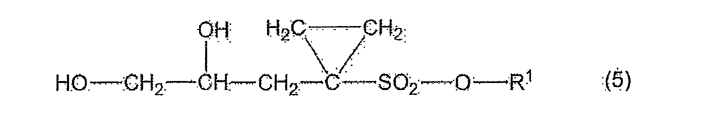 wherein R¹ represents a C₄ to C₆ branched alkyl group, with a 2,2-dialkoxypropane represented by formula (51): 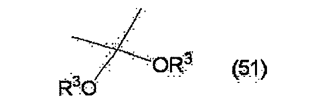 wherein R³ represents a C₁ to C₆ alkyl group,
in the presence of an acid to obtain a sulfonic acid ester represented by formula (6): 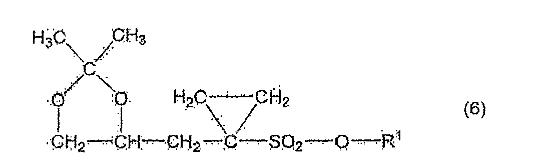 wherein R¹ is defined above,
and
(Step E)
a step of reacting the sulfonic acid ester represented by formula (6) with sodium iodide or sodium hydroxide to obtain the sulfonate represented by formula (7) :

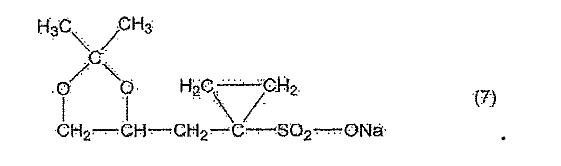

3. The method for producing the sulfonate according to claim 1, which comprises
(Step B)
a step of reacting a sulfonic acid ester represented by formula (2):
X¹- (CH₂) ₃-SO₂-O-R¹ (2)
wherein X¹ represents a chlorine atom, a bromine atom or an iodine atom, and R¹ represents a C₄ to C₆ branched alkyl group,
with an epoxy compound represented by formula (31): 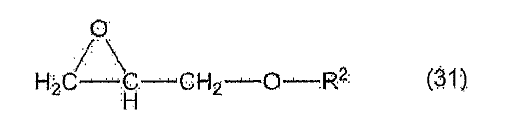 wherein R² represents a protecting group of a hydroxyl group, in the presence of a base to obtain a sulfonic acid ester represented by formula (4): 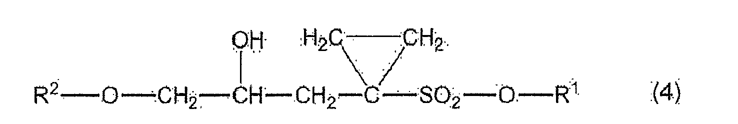 wherein R¹ and R² are as defined above,
(Step C)
a step of deprotecting the sulfonic acid ester represented by formula (4) to obtain a sulfonic acid ester represented by formula (5): 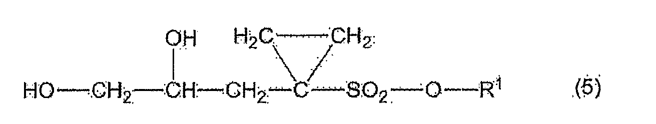 wherein R¹ is defined above,
(Step D)
a step of reacting the sulfonic acid ester represented by formula (5) with a 2, 2-dialkoxypropane represented by formula (51): 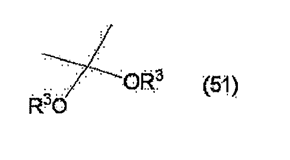 wherein R³ represents a C₁ to C₆ alkyl group,
in the presence of an acid to obtain a sulfonic acid ester represented by formula (6) 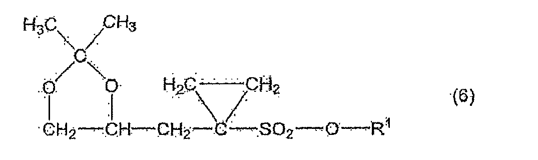 wherein R¹ is defined above,
and
(Step E)
a step of reacting the sulfonic acid ester represented by formula (6) with sodium iodide or sodium hydroxide to obtain the sulfonate represented by formula (7) :

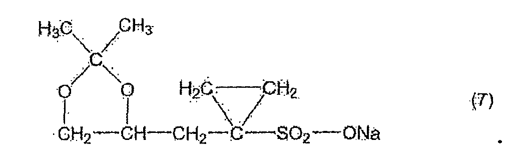

4. The method for producing the sulfonate according to claim 1, which comprises:
(Step B'-1)
a step of reacting a sulfonic acid ester represented by formula (2):
**X¹- (CH₂)₃-SO₂-O-R¹** (2)
wherein X¹ represents a chlorine atom, a bromine atom or an iodine atom, and R¹ represents a C₄ to C₆ branched alkyl group, with a base to obtain a cyclopropanesulfonic acid ester represented by formula (3): 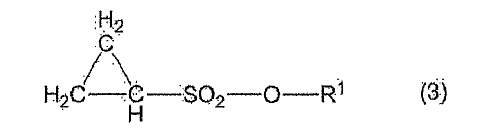 wherein R¹ is defined above,
(Step B'-2)
a step of reacting the cyclopropanesulfonic acid ester represented by formula (3) with an epoxy compound represented by formula (31): 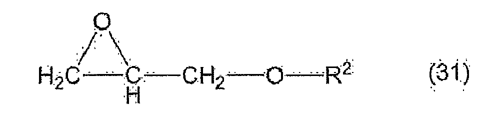 wherein R² represents a protecting group of a hydroxyl group, in the presence of a base to obtain a sulfonic acid ester represented by formula (4): 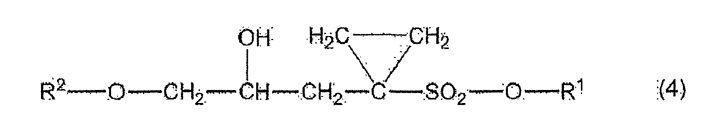 wherein R¹ and R² are as defined above,
(Step C)
a step of deprotecting the sulfonic acid ester represented by formula (4) to obtain a sulfonic acid ester represented by formula (5): 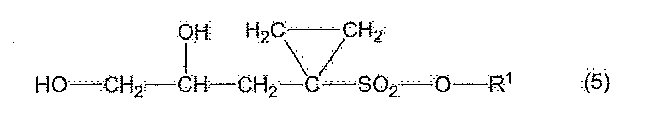 wherein R¹ is defined above,
(Step D)
a step of reacting the sulfonic acid ester represented by formula (5) with a 2,2-dialkoxypropane represented by formula (51): 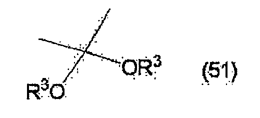 wherein R³ represents a C₁ to C₆ alkyl group,
in the presence of an acid to obtain a sulfonic acid ester represented by formula (6): 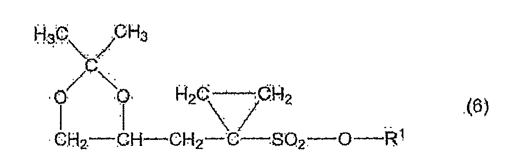 wherein R¹ is defined above,
and
(Step E)
a step of reacting the sulfonic acid ester represented by formula (6) with sodium iodide or sodium hydroxide to obtain the sulfonate represented by formula (7):

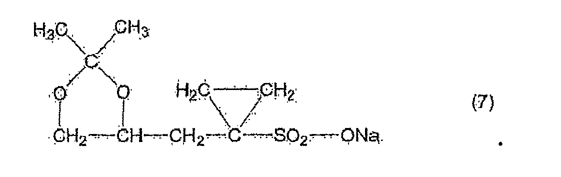

5. The method for producing the sulfonate according to claim 1, which comprises:
(Step A)
a step of reacting a sulfonyl halide represented by formula (1) :
**X¹- (CH₂)₃-SO₂-X²** **(1)**
wherein X¹ represents a chlorine atom, a bromine atom or an iodine atom,
with an alcohol represented by formula (11):
**R¹-OH** **(11)**
wherein R¹ represents a C₄ to C₆ branched alkyl group,
in the presence of a base to obtain a sulfonic acid ester represented by formula (2):
**X¹- (CH₂)₃-SO₂-O-R¹** **(2)**
wherein X¹ represents a chlorine atom, a bromine atom or an iodine atom, and R¹ is defined above,
(Step B)
a step of reacting the sulfonic acid ester represented by formula (2) with a base to obtain a cyclopropanesulfonic acid ester represented by formula (3): 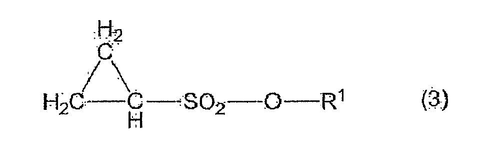 wherein R¹ is defined above (Step B'-1)
and then
reacting the cyclopropanesulfonic acid ester represented by formula (3) with an epoxy compound represented by formula (31): 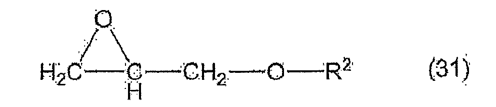 wherein R² represents a protecting group of a hydroxyl group,
in the presence of a base to obtain a sulfonic acid ester represented by formula (4): 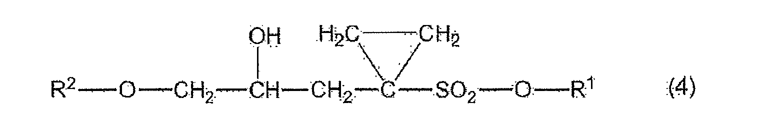 wherein R¹ and R² are as defined above (Step B'-2),
or
a step of reacting the sulfonic acid ester represented by formula (2) with the epoxy compound represented by formula (31) in the presence of a base to obtain the sulfonic acid ester represented by formula (4)
(Step C)
a step of deprotecting the sulfonic acid ester represented by formula (4) to obtain a sulfonic acid ester represented by formula (5): 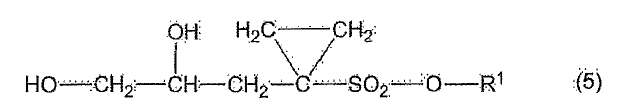 wherein R¹ is defined above,
(Step D)
a step of reacting the sulfonic acid ester represented by formula (5) with a 2,2-dialkoxypropane represented by formula (51): 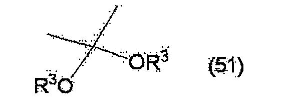 wherein R³ represents a C₁ to C₆ alkyl group,
in the presence of an acid to obtain a sulfonic acid ester
represented by formula (6): 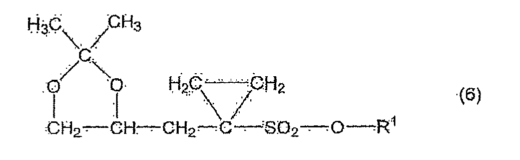 wherein R¹ is defined above,
and
(Step E)
a step of reacting the sulfonic acid ester represented by formula (6) with sodium iodide or sodium hydroxide to obtain the sulfonate represented by formula (7) :

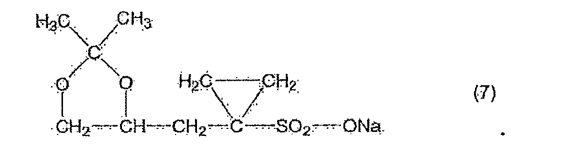

6. A sulfonic acid ester represented by formula (6): 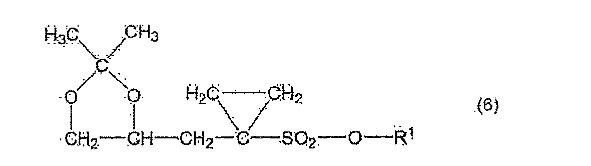 wherein R¹ represents a C₄ to C₆ branched alkyl group.

7. A sulfonic acid ester represented by formula (5): 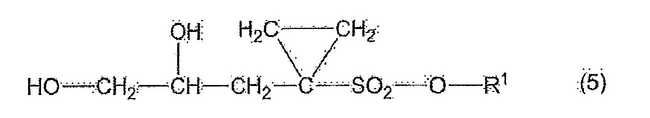 wherein R¹ represents a C₄ to C₆ branched alkyl group.

8. A sulfonic acid ester represented by formula (4): 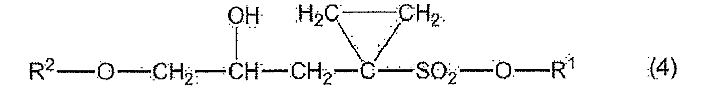 wherein R¹ represents a C₄ to C₆ branched alkyl group, and R² represents a protecting group of a hydroxyl group.

9. A cyclopropanesulfonic acid ester represented by formula (3): 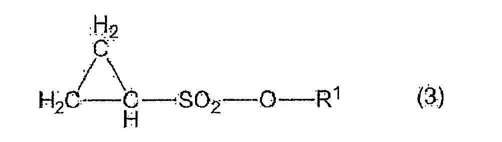 wherein R¹ represents a C₄ to C₆ branched alkyl group.

10. A sulfonic acid ester represented by formula (2):
X¹- (CH₂) ₃-SO₂-O-R¹ (2)
wherein X¹ represents a chlorine atom, a bromine atom or an iodine atom, and R¹ represents a C₄ to C₆ branched alkyl group.
